# EUROPEAN PATENT SPECIFICATION

(11) **EP 1 842 524 B1**
(45) Date of publication and mention of the grant of the patent: **23.01.2013**
(21) Application number: 05816792.5
(22) Date of filing: 13.12.2005
(51) Int. Cl.: A61K 8/81, B82Y 5/00, A61K 8/19, A61Q 11/00

(54) **CLEANING LIQUID FOR ORAL CAVITY**
REINIGUNGSFLÜSSIGKEIT FÜR DIE MUNDHÖHLE
LIQUIDE NETTOYANT POUR CAVITE BUCCALE

(30) Priority: 13.12.2004 JP 2004359639
(43) Date of publication of application: 10.10.2007
(73) Proprietor: APT Co., Ltd., Shibuya-ku, Tokyo 151-0053 (JP); Dentalarrow Co., Ltd., Sapporo-shi, Hokkaido, 0010023 (JP)
(72) Inventor: OKAYAMA, Minenobu, Toride-shi, Ibaraki 302-0034 (JP); KOSHIRO, Kenichi, Sapporo-shi, Hokkaido 065-0021 (JP)
(74) Representative: Polypatent
(86) International application number: PCT/JP2005/022839
(87) International publication number: WO 2006/064788

(56) References cited:
- EP-A1- 1 550 637
- EP-A1- 1 598 071
- EP-A1- 1 598 072
- WO-A-2004/073722
- WO-A-2004/073723
- WO-A1-2004/039735
- WO-A2-02/085385
- JP-A- 06 122 617
- JP-A- 11 060 493
- JP-A- 2001 010 954
- JP-A- 2002 193 777
- JP-A- 2004 161 632
- JP-A- 2004 525 980
- YAHAGI K.: 'Yakuyo Hamigakizai Senkozai Kochu Seiryozai no Kenkyu Kaihatsu no Genjo to Kadai.(Current aspects and prospective problems in research and development for medicated dentifrice, mouthwash, and oral freshner.)' FRAGRANCE JOURNAL vol. 28, no. 1, 2000, pages 89 - 95, XP002996583

## Description

### Field of the Invention

The present invention relates to an oral wash. More specifically, the present invention relates to an oral wash that is capable of conveniently and quickly eliminate reactive oxygen species in the oral cavity.

### Background Art

Periodontal diseases, as typical diseases in the oral cavity, causes inflammation due to infection with pathogenic bacteria, and reactive oxygen species produced in the lesion give oxidation stresses. In periodontal diseases, the actions of inflammation mediators are piled on the oxidation stress to cause destructions of gingival tissues and alveolar bone, and therefore, the diseases constitute a major cause of tooth loss. In recent years, bleaching treatment of colored or discolored teeth has been applied in the dental field from a cosmetic viewpoint. The bleaching of teeth is performed by contacting a bleaching agent such as hydrogen peroxide, urea peroxide and hypochlorous acid with surfaces of teeth and maintaining the contact for a certain period of time. Since all the aforementioned bleaching agents generate free radicals such as reactive oxygen species, they may damage the gingiva and mucosa of the oral cavity.

As a composition for eliminating reactive oxygen species in the oral cavity, a composition for oral cavity is disclosed in Japanese Patent No. 3582537, in which reactive oxygen eliminating effect and mucosal absorption of magnesium ascorbyl phosphate are improved. This publication teaches that, when magnesium ascorbyl phosphate and eugenol are used in combination, or they are used further in combination with menthol, gingivitis is more effectively improved. Japanese Patent Unexamined Publication (KOKAI) No. 2004-231602 discloses a composition for oral cavity containing a galenical extract having free radical elimination activity and having an anti-oxidation action. This composition preferably contains coenzyme Q10, and can be used for gingival massage. Japanese Patent Unexamined Publication No. 2003-176225 discloses a composition for oral cavity containing one or more kinds of extracts of plants belonging to the family *Gramineae,* genus *Oryza,* the family *Myricaceae,* genus *Myrica* and the like. However, these publications contain no description suggesting that a transition metal is effective for eliminating reactive oxygen species in the oral cavity.

Japanese Patent Unexamined Publication No. 2004-107231 discloses a composition for oral cavity containing a free radical eliminating substance for protecting gingiva or mucosa in the oral cavity from a bleaching treatment with a tooth bleaching agent. As the free radical eliminating substance, an enzyme type antioxidant or a non-enzyme type antioxidant is typically used, and specifically exemplified are enzyme type antioxidants such as catalase, superoxide dismutase (SOD), glutathione peroxidase, glutathione-S-transferase, and peroxidase, and non-enzyme type antioxidants such as vitamin E, vitamin C, glutathione, carotinoid, flavonoid, ubiquinone, γ-orizanol, metallothionein, sodium sulfite, citric acid, cysteine, saccharides, deferoxamine, uric acid, ceruloplasmin, transferrin, ferritin, haptoglobin, albumin, bilirubin, pyruvic acid, lecithin, thioglycolic acid, thiomalic acid, sodium nitrite, sodium edetate, and hydroquinone. However, this publication does not teach a transition metal, per se, as an antioxidant.

WO 02/08 5385 discloses the use of antimicrobial noble metals for treating disorders of the mucosal membranes.

International Publication WO2004/73723 discloses a medicament for prophylactic and/or therapeutic treatment of neurodegenerative diseases such as amyotrophic lateral sclerosis, rheumatic diseases, ischemic heart diseases, stress ulcer, dermatitis, arteriosclerosis, and hyperlipidemia, which comprises fine particles of a noble metal. However, this publication does not suggest nor teach that the noble metal fine particles as the active ingredient of the aforementioned medicament are effective as an active ingredient of an oral wash.

### Disclosure of the Invention

### Object to be Achieved by the Invention

An object of the present invention is to provide an oral wash which is capable of conveniently and quickly eliminate reactive oxygen species that cause damages of mucosa in the oral cavity and periodontal tissue.

### Means for Achieving the Object

The inventors of the present invention conducted various researches to achieve the aforementioned object, and as a result, they found that when an oral wash containing fine particles of a noble metal consisting of platinum colloid, in an aqueous medium was used, reactive oxygen species in the oral cavity was very efficiently and conveniently eliminatable. The present invention was achieved on the basis of the aforementioned finding.

The present invention provides an oral wash for use in the prophylactic and/or therapeutic treatment of periodontal diseases, endermosis or stomatitis in the oral cavity which are caused by reactive oxygen species comprising an aqueous dispersion of noble metal fine particles consisting of platinum colloid having a mean particle size of 10 nm or smaller, and a water-soluble polymer.

### Brief Description of the Drawing

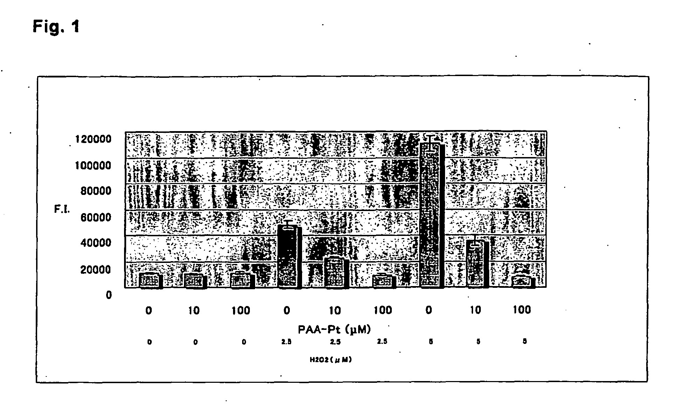

[Fig. 1] Fig. 1 shows the action of the oral wash of the present invention. Best Mode for Carrying Out the Invention

As the noble metal fine particles, fine particles that have a large specific surface area and can form a colloidal state of superior surface reactivity are preferred. Fine particles having a mean particle size of 10 nm or smaller most preferably about 1 to 6 nm, are used.

Various preparation methods for transition metal fine particles, preferably noble metal fine particles, are known (for example, Japanese Patent Publication (KOKOKU) Nos. 57-43125, 59-120249, Japanese Patent Unexamined Publication Nos. 9-225317, 10-176207, 2001-79382, 2001-122723 and the like), and those skilled in the art can easily prepare the fine particles by referring to these methods. For example, as the method for producing noble metal fine particles, a chemical method called precipitation method or metal salt reduction method, a physical method called combustion method and the like can be used. Fineparticles prepared by any of the methods may be used as the active ingredient of the mouth wash of the present invention. It is preferable to use fine particles prepared by the metal salt reduction method from viewpoints of easiness of the production and quality of the fine particles. Methods for preparing noble metal fine particles as a preferred embodiment of the present invention will be described below.

In the metal salt reduction method, for example, an aqueous solution or organic solvent solution of a water-soluble or organic solvent-soluble noble metal salt or noble metal complex is prepared, then a water-soluble polymer is added to the solution and pH of the solution is adjusted to 9 to 11, and the solution can be refluxed by heating in an inert atmosphere to reduce the metal salt or metal complex to obtain metal fineparticles. Types of the water-soluble or organic solvent-soluble noble metal salt are not particularly limited. For example, acetate, chloride, sulfate, nitrate, sulfonate, phosphate and the like can be used, and complexes thereof may also be used.

Types of the water-soluble polymer used for the metal salt reduction method are not particularly limited. For example, polyvinylpyrrolidone, polyvinyl alcohol, polyacrylic acid, cyclodextrin, amylopectin, methylcellulose and the like can be used, and two or more kinds of these polymers may be used in combination. Polyvinylpyrrolidone can be preferably used, and poly(1-vinyl-2-pyrrolidone) can be more preferably used. It is also possible to use various kinds of surface active agents such as anionic, nonionic or liposoluble surface active agents instead of the water-soluble polymer or together with the water-soluble polymer. When an alcohol is used to perform the reduction, ethyl alcohol, n-propyl alcohol, n-butyl alcohol, n-amyl alcohol, ethylene glycol or the like is used, and an organic acid or the like is used. However, the methods for preparing noble metal fine particles are not limited to the methods explained above.

As the oral wash of the present invention, an aqueous dispersion comprising platinum fine particles, in a colloidal state according to present claim 1 prepared by the aforementioned methods, per se, may be used. An aqueous suspension in which the noble metal fine particles, associate to form clusters may also be used as the oral wash of the present invention. The dispersion medium of the aqueous suspension of the noble metal fine particles according to claim 1 of the present invention consists preferably of substantially water alone. The medium contains one or more kinds of the aforementioned water-soluble polymers. The medium may contain surface active agents and the like used for the preparation of the fineparticles in a colloidal state. The medium may also contain a small volume of a water-miscible organic solvent such as ethanol and glycerol in such a range that stable dispersion of the noble metal fine particles, is not destroyed and the dispersion is acceptable as an oral wash.

A concentration of noble metal fine particles, contained in the oral wash of the present invention is not particularly limited. The concentration is, for example, about 0.1 to 100 µM, preferably about 0.5 to 10 µM, most preferably about 1 to 5 µM. When a sufficient effect is desired with application for a short period of time or application in a small volume, a concentration not lower than 10 µM is preferred. The oral wash of the present invention may contain one or more kinds of ordinarily used pharmaceutical additives in addition to platinum fine particles as the active ingredient. For example, one or more kinds of pH modifiers, buffering agents, preservatives, isotonic agents, thickeners, corrigents such as sweeteners, flavoring agents, colorants, and the like may be contained. For example, by adding an appropriate thickener to the oral wash of the present invention to impart appropriate viscosity, it becomes possible to make the oral wash containing the transition metal fine particles of a low concentration exhibit prolonged action.

The oral wash of the present invention is capable of very efficiently eliminate reactive oxygen species in the oral cavity by a convenient method for use, and therefore, is useful for prophylactic and/or therapeutic treatment of periodontal diseases, endermosis or stomatitis in the oral cavity, which are caused by reactive oxygen species. Examples of active oxygen species removable with the oral wash of the present invention include, for example, superoxide anions, hydroxy radicals, singlet oxygens, hydrogen peroxide, and the like, but not limited to these examples. Because the action of the platinum fine particles as the active ingredient of the oral wash of the present invention is a reductive catalyst-like action, the active oxygen removing effect is exhibited over a long period of time as long as the fine particles remain in application sites of the oral cavity.

The method for use and application site of the oral wash of the present invention are not particularly limited, and can be used by arbitrary methods for washing any sites in the oral cavity. For example, the wash can be used for washing of mucosa in the oral cavity, periodontium, tooth, tongue, and the like. Typically, an appropriate volume of the oral wash of the present invention can be kept in the mouth to rinse a site including a pathological lesion several times, and then spitting out the wash. Such embodiment is suitable for treatment of teeth, or periodontium, and odontectomy in the odontotherapy, or bleaching treatment of teeth in the field of cosmetic dentistry. The oral wash of the present invention can also be impregnated into absorbent cotton or swab, and applied to an application site in the oral cavity, or filled in a syringe or a nebulizer, and injected or sprayed on an application site. Furthermore, the oral wash of the present invention may be used, for example, instead of water for rinsing the mouth in treatments by dentists or oral surgeons, or the oral wash of the present invention may be prescribed for patients for use in rinsing several times a day in their homes.

### Examples

Hereafter, the present invention will be explained more specifically with reference to the examples. However, the scope of the present invention is not limited to the following examples.

### Example 1: Preparation of the oral wash of the present invention

In a 100-ml 2-neck pear-shaped flask connected with an Allihn condenser and a 3-neck joint, 0.1467 g of poly(1-vinyl-2-pyrrolidone) (Wako Pure Chemical Industries) was placed and dissolved in 23 ml of distilled water. This solution was stirred for 10 minutes, then mixed with 2 ml of 1.66 x 10⁻² M chloroplatinic acid solution obtained by dissolving hexachloroplatinic acid (H₂PtCl₆ · 6H₂O, Wako Pure Chemical Industries) in distilled water, and stirred for additional 30 minutes. The inside atmosphere of the reaction system was replaced with nitrogen gas. Twenty five ml of special grade ethanol was added to the reaction mixture and the resulting mixture was refluxed at a temperature of 100°C for 2 hours while the nitrogen atmosphere was maintained. An ultraviolet-visible light spectral scanning analysis of the reaction mixture was performed to confirm disappearance of the platinum ion peak and saturation of the peak due to scattering peculiar to metal solid and thereby confirm completion of the reduction reaction. The organic solvent was evaporated under reduced pressure to prepare a platinum colloidal solution containing platinum fineparticles (mean particle size: 2.4 ± 0.7 nm) (PVP-Pt solution). This platinum colloidal solution was diluted to 1 pM with deionized distilled water to prepare the oral wash of the present invention.

### Example 2: Preparation of the oral wash of the present invention

A platinum colloidal solution (PAA-Pt solution) containing platinum fine particles having a mean particle size of 2.0 ± 0.4 nm was prepared in the same manner as that in Example 1, except that poly(sodium acrylate) (Aldrich) was used in an amount of 125 times that of Pt in terms of unit weight instead of poly(1-vinyl-2-pyrrolidone). This platinum colloidal solution was diluted to 1 mM or 100 µM with distilled water for injection to prepare the oral wash of the present invention.

### Example 3: Test Example 1

The oral wash obtained in Example 2 was used to confirm the hydroxy radical suppressing effect thereof. The following solutions were prepared.
1. 0.1 M Phosphate buffer (Na₂HPO₄/NaH₂PO₄, pH 7.4)
2. 100 µM APF (aminophenylfluorescein, capable of specifically detecting reactive oxygen species such as hydroxy radicals, obtained by diluting 5 mM APF (Daiichi Pure Chemicals) with the phosphate buffer of 1. mentioned above)
3. 5 µM H₂O₂ and 10 µM H₂O₂ (obtained by diluting 9.7 M H₂O₂ with deionized distilled water)
4. 2 µM HRP (peroxidase, diluted with the phosphate buffer of 1. mentioned above)
5. 100 µM and 1 mM PAA-Pt (diluted with deionized distilled water)

A Fluoroplate produced by Nunc (96-well, #237107) was used, and (1) 20 µl of the 0.1 M phosphate buffer, (2) 50 µl of the aqueous hydrogen peroxide (0 µM: deionized distilled water as control, 5 µM aqueous hydrogen peroxide, or 10 µM aqueous hydrogen peroxide), and 10 µl of the 2 µM peroxidase solution were added to each well, and incubated at 37°C for 20 minutes under light shielding. To each solution, 10 µl of PAA-Pt (0 µM: deionized distilled water as control, 100 µM PAA-Pt, or 1 mM PAA-Pt) was added, and the mixture was incubated at 37°C for 10 minutes under light shielding. To each solution, 10 µl of the 100 µM APF solution was further added, and the mixture was incubated at 37°C for 5 minutes under light shielding. After 10 minutes, fluorescence was measured with Wallac 1420 ARVOₛₓ multilabel counter (PE) (excitation wavelength: 485 nm, measurement wavelength: 535 nm) to examine the action of PAA-Pt. The results are shown in Fig. 1. PAA-Pt significantly suppressed hydroxy radicals generated from hydrogen peroxide even at 10 µM, and it suppressed hydroxy radicals to a level similar to that of the control (without hydrogen peroxide) at 100 µM.

### Test Example 2

Patients were made to use the oral wash obtained in Example 2 for 14 days (3 times a day, 7 to 10 ml for each time), and the patients and medical practitioners were interviewed by using interview sheets to know what kind of change arose in the oral cavity. The patients consisted of those under treatment of periodontal diseases (67%), those under maintenance (17%), those scheduled or undergone a surgical operation (8%), and those under general restoration or prosthesis (8%). As a result, answers of observing any changes were obtained from 65% of the patients, and 58% of the patients judged that the wash was effective. Moreover, 50% of the medical practitioners judged that the use of the wash was effective (excellent wash: 33%, rather good wash: 25%). In particular, improvements of subjective symptoms were reported, for example, reduced bleeding upon use of toothbrushes (23%), reduced onset of stomatitis or ready curability of stomatitis (8%), reduced month dryness (38%), reduced pain of the gingiva (15%) and the like.

### Industrial Applicability

Use of the oral wash of the present invention achieves extreme quick and convenient elimination of active oxygen species, which causes inflammation of parodontium, mucosa in the oral cavity, and thus is capable of effectively preventing periodontal diseases, endermosis or stomatitis in the oral cavity which are caused by reactive oxygen species.

## Claims

1. An oral wash for use in the prophylactic and/or therapeutic treatment of periodontal diseases, endermosis or stomatitis in the oral cavity which are caused by reactive oxygen species comprising an aqueous dispersion of noble metal fine particles consisting of platinum colloid having a mean particle size of 10 nm or smaller, and a water-soluble polymer.

## Patentansprüche

1. Ein Mundspülmittel zur Verwendung in der vorbeugenden und/oder therapeutischen Behandlung von Erkrankungen des Zahnfleisches, Endermose oder Mundschleimhautentzündung in der Mundhöhle, die durch reaktive Sauerstoffspezies verursacht werden, enthaltend eine wässrige Dispersion von Edelmetallfeinpartikeln bestehend aus Patinkolloid mit einer durchschnittlichen Partikelgröße von 10 nm oder kleiner und einem wasserlöslichen Polymer.

## Revendications

1. Bain de bouche pour son utilisation dans un traitement prophylactique et/ou thérapeutique des maladies parodontales, de l'endermose ou de la stomatite dans la cavité buccale, qui sont provoquées par des dérivés réactifs de l'oxygène, comprenant une dispersion aqueuse des fines particules en métal noble consistant en colloïde de platine ayant une taille moyenne des particules de 10 nm ou plus petites et un polymère soluble dans l'eau.
